# EUROPEAN PATENT SPECIFICATION

(11) **EP 1 939 172 B1**
(45) Date of publication and mention of the grant of the patent: **11.07.2012**
(21) Application number: 06807907.8
(22) Date of filing: 03.08.2006
(51) Int. Cl.: C07C 269/04, C07C 271/44, C07C 215/50, C07C 217/56

(54) **METHOD OF OBTAINING PHENYL CARBAMATES**
VERFAHREN ZUR GEWINNUNG VON CARBAMINSÄUREPHENYLESTERN
PROCEDE D'OBTENTION DE CARBAMATES DE PHENYLE

(30) Priority: 04.08.2005 ES 200501969
(43) Date of publication of application: 02.07.2008
(73) Proprietor: Interquim, S.A., 08173 Sant Cugat del Vallés, Barcelona (ES)
(72) Inventor: MURILLO GARRIDO, José Vicente Ragactives, S.L., 47151 Boecillo - Valladolid (ES); ARMENGOL MONTSERRAT, Miquel Interquim, S.A., E-08173 Sant Cugat del Vallés- Barcelona (ES); MARTÍN JUÁREZ, Jorge Ragactives, S.L., E-47151 Boecillo- Valladolid (ES)
(74) Representative: ABG Patentes, S.L.
(86) International application number: PCT/ES2006/000459
(87) International publication number: WO 2007/014973

(56) References cited:
- WO-A1-2004/037771
- WO-A1-2004/037771
- WO-A1-2006/048720
- WO-A1-2006/068386
- WO-A1-2006/068386
- US-A- 3 457 266
- PHILIP J C ET AL: "The resolution of alpha-m-hydroxy-phenylethylmethylamine and the preparation of d- and l-miotine (methylurethanes of d- and l-alpha-m-hydroxyphenylethyl-dimethylamine )", JOURNAL OF THE CHEMICAL SOCIETY, CHEMICAL SOCIETY, LETCHWORTH; GB, 1 January 1932 (1932-01-01), pages 2513-2518, XP9034872, ISSN: 0368-1769
- DATABASE CAPLUS [Online] CHEMICAL ABSTRACTS SERVICE, COLUMBUS, OHIO, US; 2007, RAO, RAMAKRISHNA ET AL: "A process and intermediate product for the production of rivastigmine", XP002620899, retrieved from STN Database accession no. 2007:631990
- CISZEWSKA G. ET AL.: 'Synthesis of tritium, deuterium and carbon-14 labeled (S)-N-ethyl-N- methyl-3-[1-(dimethyl-amino)ethyl]carbamic acid, phenyl ester, (L)-dihydroxy-butanedioic acid salt (SDZ ENA 713 hta), an ivestigational drug for the treatment of Alzheimer's disease' J.LABELLED COMPS vol. 39, no. 8, 1997, pages 651 - 668, XP002269029
- MCL. MACDONALD J. ET AL.: 'The resolution of alpha-m-hydroxy-phenylethylmethylamine and the preparation of d- and l-miotine (methylurethanes of d- and l-alpha-m-hydroxyphenylethyl-dimethylamine) ' J. CHEM. SOC. 1932, pages 2513 - 2518, XP008126318
- CHEN C-P. ET AL.: 'A general enantioselective synthesis of alpha-arylethylamines' TETRAPHEDRON LETT. vol. 32, no. 49, 1991, pages 7175 - 7178, XP009025296
- GRETHE G. ET AL.: 'Syntheses in the isoquinoline series. Synthesis of 2,3-dihydro-4(H)- isoquinolones' J. ORG. CHEM. vol. 32, no. 2, 1968, pages 491 - 494, XP001181465

## Description

### Field of the Invention

The invention relates to a method of obtaining phenyl carbamates, their enantiomers or mixtures thereof, or their pharmaceutically acceptable salts, as well as an intermediate useful for preparing said phenyl carbamates.

### Background of the Invention

Some phenyl carbamate derivatives, and quite particularly the compound Rivastigmine, the International Nonproprietary Name for the compound (*S*)-*N*-ethyl-3-[1-(dimethylamino)ethyl]-*N-*methyl-phenylcarbamate, are compounds with exceptional cholinesterase inhibitor activity in the central nervous system, whereby prolonging the life of cetylcholine and improving cholinergic transmission. These compounds have proven to be particularly useful in the treatment of neurodegenerative diseases, and particularly in the treatment of senile dementia and Alzheimer's disease. In Czech patent application PV 1991-4110, it is further proven by means of *in vitro* and *in vivo* experiments that the active (S) isomer is a much more effective and selective cholinesterase inhibitor than the racemic mixture of the two isomers.

Rivastigmine and other phenyl carbamates were first described in Israeli patent IL 74497 (EP 193926). Said application describes obtaining Rivastigmine by means of the amidation reaction of 3-[1-(dimethylamino)ethyl] hydroxyphenyl with an isocyanate or with a carbamoyl halide. However, this patent does not mention any possibility of converting the racemic mixture obtained into its optically active enantiomers.

The aforementioned Czech patent application PV 1991-4110 describes a method of preparing the Rivastigmine (S) enantiomer from the racemic mixture consisting of preparing the diastereoisomeric salts with (+)-0,0-di-(p-toluoyl)-D-tartaric acid and the separation thereof by means of crystallization. The Rivastigmine (S) enantiomer is separated from the obtained salt with a sodium hydroxide solution.

International patent application WO03/101917 describes obtaining Rivastigmine and other phenyl carbamates by means of reacting 3-(1-dimethylamino ethyl) phenol with N-ethyl-N-methyl-4-nitrophenyl carbamate and subsequently resolving the racemic mixture with (+)-*p*-toluoyl tartaric acid monohydrate to obtain the (S) enantiomer of therapeutic interest.

As described in PV 1991-4110 and in WO03/101917, the main technological drawback involved in resolving the racemic mixture in the last step of synthesis is based on the fact that 50% of the prepared racemic product, i.e. the (R) enantiomer, is lost in that step. In fact, this loss is greater given that optical resolution never separates the enantiomers quantitatively, an additional recrystallization step being required. This causes the total synthesis yield, as well as the cost-effectiveness of the process, to be low.

An alternative method of obtaining Rivastigmine which seeks to overcome the aforementioned drawback is described in international patent application WO2004/037771. Like the method described in IL74497, this method comprises the amidation reaction of 3-[1-(dimethylamino)ethyl] phenol with a carbamoyl halide, although in this case, the amidation reaction is carried out on the (S) enantiomer of said 3-[1-(dimethylamino)ethyl]phenol compound, which has been previously separated by resolution. It is thus not necessary to resolve the racemic mixture in the last step of the synthetic sequence. Nevertheless, the main drawback of this synthesis continues to be the step of resolving the enantiomeric mixture given that, despite performing a previous step, the obtained yield is only 25%.

It is therefore necessary to solve the problems associated with the methods belonging to the state of the art and to provide an alternative method of obtaining Rivastigmine and other phenyl carbamates which improves the cost-effectiveness of the process by increasing the yield of their synthesis and allowing to quantitatively obtain the (S) enantiomer.

### Summary of the Invention

The invention faces the problem of providing an alternative method of obtaining phenyl carbamates, and particularly Rivastigmine, which overcomes the problems existing in the different aforementioned state of the art syntheses.

The solution provided by the invention is based on the fact that the inventors have observed that it is possible to obtain phenyl carbamates, their solvates, hydrates or pharmaceutically acceptable salts from a compound of general formula (IV) (defined below) with a carbamoyl halide of formula (III) (defined below) in the presence of a base, by means of a nucleophilic substitution reaction and subsequent reductive amination of the ethylamino group. Said compound of general formula (IV) can be obtained from inexpensive commercial compounds.

A method such as the method provided by the present invention has the advantage of being able to resolve the enantiomeric mixture in a prior step of the synthetic route obtaining the desired enantiomer with a good yield and high quality. All this contributes to reducing the global cost of the method, making said method to be commercially interesting and allowing it to be put into practice on an industrial level.

Therefore, in one aspect the invention relates to a method of obtaining phenyl carbamates of general formula (I) (defined below) which comprises reacting a compound of formula (IV) with a compound of formula (III) to give a compound of formula (II), and then subjecting said compound of formula (II) to a reductive amination reaction, or alternatively to a methylation reaction by reacting it with a methyl halide, to obtain the compound of formula (I).

Said compound of formula (II), useful for obtaining the compound of formula (I), is an additional aspect of the present invention.

In another aspect, the invention relates to a method of obtaining phenyl carbamates of formula (I) from a compound of formula (VII) (defined below) which, by means of reductive amination with methylamine in the presence of a reducing agent, becomes a compound of formula (VI), which is subjected to a demethylation reaction to obtain a compound of formula (V) which, by resolution, yields the enantiomer of formula (IV), from which the compound of formula (I) is obtained by the previously defined method.

In another aspect, the invention relates to an alternative method of obtaining phenyl carbamates of formula (I) which comprises subjecting a compound of formula (VI) to a resolution process to obtain an enantiomer of formula (VIa) (defined below), from which the enantiomer of formula (IV) is directly obtained by demethylation, and from this compound, the compound of formula (I) is obtained by the previously defined method.

In another aspect, the invention relates to a method for resolving the enantiomeric mixture of the compound of formula (VI) by means of fractional crystallization of diastereoisomeric salts obtained by reacting the corresponding enantiomers of the enantiomeric mixture with a chiral acid, subsequently recrystallizing to obtain the diastereoisomeric salt of the desired enantiomer and obtaining said enantiomer by resuspending said salt in an aqueous-organic medium, neutralizing the medium and isolating the enantiomer in the form of a base or in the form of an acid addition salt. In a particular embodiment, the isolated enantiomer is the compound of formula (VIa), which can be used in the synthesis of phenyl carbamates of formula (I). Said compound of formula (VIa), as well as obtaining it by resolving an enantiomeric mixture of the compound of formula (VI), are additional aspects of this invention.

### Detailed Description of the Invention

In one aspect, the invention relates to a method, hereinafter method of the invention [1], of obtaining a phenyl carbamate of general formula (I): 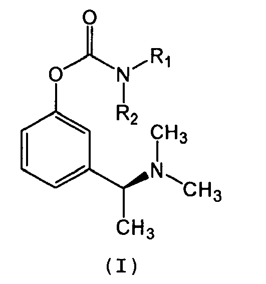 wherein
R₁ is C₁-C₅ lower alkyl or benzyl; and
R₂ is methyl, ethyl or propyl;
its solvates, hydrates or pharmaceutically acceptable salts,
which comprises:
a) reacting a compound of formula (IV): 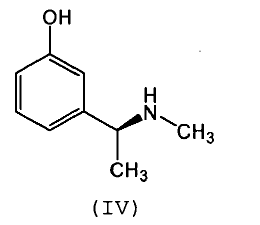 in the presence of a base, with a compound of formula (III) : 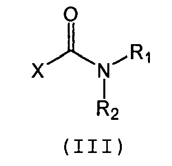 wherein R₁ and R₂ have the previously mentioned meanings and X is a halogen,
   to give a compound of formula (II): 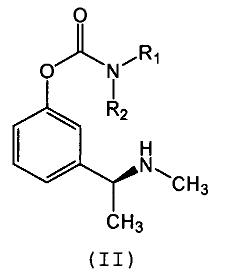 wherein R₁ and R₂ have the previously mentioned meanings; and
b) subjecting said compound of formula (II) to a reductive amination reaction, or alternatively to a methylation reaction by reacting said compound of formula (II) with a methyl halide, to obtain the compound of formula (I).

The reaction between the enantiomer of formula (IV) and the carbamoyl halide [compound of formula (III)] defined in synthesis step a) is a nucleophilic substitution reaction which allows forming the carbamate group of the compound of formula (I). This reaction is carried out in the presence of a base which abstracts the phenol proton, giving the salt that is formed a higher nucleophilic character. Unlike what was expected, the inventors have surprisingly achieved reacting said salt with the carbamoyl halide in a chemoselective manner without there being competition by the monomethylated amine present in the molecule, therefore maintaining the optical isomerism of the starting product in the obtained product.

Virtually any base can be used in this nucleophilic substitution reaction; nevertheless, in a particular embodiment said base is an organic or inorganic base that can completely abstract the proton from the phenol to give the corresponding salt or phenoxide, such as NaOH, KOH, t-BuOK, NaH or NaOMe for example, preferably NaH or t-BuOK.

The nucleophilic substitution reaction is carried out in any solvent that is considered suitable. In a particular embodiment non-protic solvents such as ethers, dichloromethane, toluene or dipolar aprotic solvents, for example, dimethylformamide (DMF) or dimethylsulfoxide (DMSO), preferably tetrahydrofuran (THF), are used. This reaction is carried out at a temperature comprised between -20°C and 20°C, preferably between -5°C and 5°C. Said nucleophilic substitution reaction takes place with a high yield, comprised generally between 70% and 100%, typically between 85% and 95%, thus contributing to the high global yield of the method of obtaining the compound of formula (I) provided by this invention.

The second step of the method of the invention [1] [step b)] consists of methylating the primary amine (compound of formula (II)), which can be carried out by any of the methods known by a person skilled in the art. By way of illustration, said step can be carried out by subjecting the compound of formula (II) to a reductive amination reaction, or alternatively by reacting said compound of formula (II) with a methyl halide.

In a particular embodiment, step b) is carried out by means of a reductive amination reaction by reacting the compound of formula (II) with formaldehyde in the presence of a reducing agent and an acid. Virtually any suitable reducing agent can be used; nevertheless, in a particular embodiment a hydride, such as sodium cyanoborohydride, sodium borohydride along with Ti(OiPr)₄, etc., can be used, among others, as a reducing agent. An organic acid such as formic acid, acetic acid, etc., can be used as an acid. In a preferred embodiment of the present invention, formaldehyde, sodium cyanoborohydride and acetic acid are used. Alternatively, reductive amination can be carried out, if desired, using only formic acid, which can also act as a reducing agent. Alcohols, for example, alcohols with one to five carbon atoms [C₁-C₅], such as methanol, ethanol and isopropanol, preferably methanol, can be used as the solvent, although other alcohols can also be used. The reaction is carried out at a temperature comprised between 20°C and 40°C, preferably between 15°C and 30°C.

In another particular embodiment, step b) is carried out by means of a methylation reaction which comprises reacting the compound of formula (II) with a methyl halide. Although virtually any methyl halide can be used, methyl bromide or methyl iodide is used in a preferred embodiment. The solvents used in this reaction can be, among others, acetone, toluene, DMF, DMSO, acetonitrile, dichloromethane, ethyl acetate or alcohols in general; acetone, acetonitrile or dichloromethane, preferably acetone, is advantageously used. The reaction is carried out at a temperature comprised between 0°C and 30°C, preferably between 15°C and 25°C.

The compound of formula (IV) can be obtained by means of the synthesis reaction sequence shown in Reaction Scheme I.

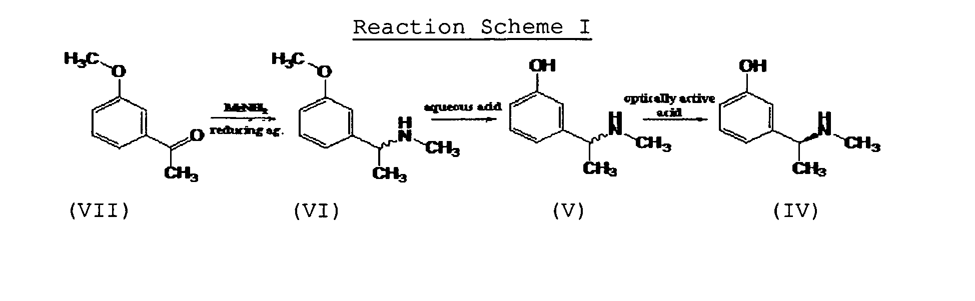

These steps will be described in detail below. Nevertheless, as can be seen, the starting compound of the method of the invention [1] [compound of formula (IV)] can be obtained by resolving the enantiomeric mixture containing it with a high yield, which is a significant advantage. Although there is no desire to be related to any theory, it is thought that resolving the compound of formula (IV), in which the amine is monoalkylated, is easier and more efficient than resolving similar compounds such as the one used in the state of the art in which the amine is dialkylated.

Alternatively, the compound of formula (IV) can be obtained by means of the synthesis reaction sequence shown in Reaction Scheme II.

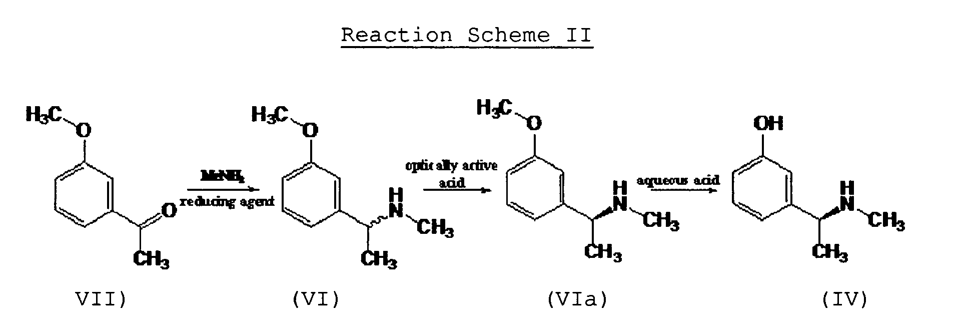

These steps will also be described in detail below. Unlike the synthesis sequence shown in Reaction Scheme I, the enantiomeric mixture is resolved on the methoxylated substrate [compound of formula (VI)] to give rise to the corresponding desired enantiomer [compound of formula (VIa)] with very good yields.

The method of the invention [1] provides compounds of formula (I). In a particular embodiment, said method provides a compound of formula (I) in which R₁ is methyl and R₂ is ethyl, i.e. Rivastigmine, as well as its pharmaceutically acceptable salts.

The compound of formula (I) is an amine and can form addition salts with organic or inorganic acids when it reacts with a stoichiometric amount of the suitable acid in water, in an organic solvent or in a mixture of both. Normally the preferred non-aqueous mediums are ether, ethyl acetate, ethanol, isopropanol or acetonitrile. The acid addition salts include the addition salts of mineral acids, such as hydrochloride, hydrobromide, hydroiodide, sulfate, nitrate and phosphate, and organic acid addition salts such as acetate, maleate, fumarate, lactate, citrate, oxalate, succinate, tartrate, malate, mandelate, methanesulfonate and p-toluenesulfonate. Said salts can be obtained by conventional methods by reacting the free amine with the acid in question. In a particular embodiment, said salt is a pharmaceutically acceptable salt, for example tartrate. Said salt can be obtained by reacting the free amine with tartaric acid. Said addition salt can optionally be transformed into the corresponding free amine by conventional methods if desired, for example by changing the pH of a solution comprising said salt until obtaining the free amine.

The compound of formula (I) can be obtained in free base or in salt form. In both cases it is preferably obtained in crystalline form, both as free compounds and as solvates (for example hydrates), both forms being included in the scope of the present invention. The solvation methods are generally known in the state of the art.

In another aspect, the invention relates to a compound of formula (II): 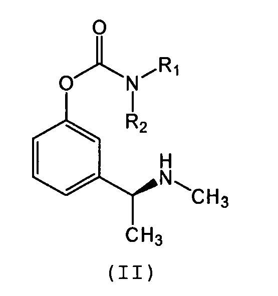 wherein
R₁ is C₁-C₅ lower alkyl or benzyl;
R₂ is methyl, ethyl or propyl;
or its salts.

The compound of formula (II) can be obtained according to the method previously described in the method of the invention [1] from the compound of formula (IV) by reacting with the compound of formula (III). Said compound of formula (II) can be used to obtain the compound of formula (I). In a particular embodiment, in the compound of formula (II) R₁ is methyl and R₂ is ethyl, i.e. the compound of formula (II) is L-(S)-N-ethyl-3-[1-(methylamino)ethyl]-N-methyl-phenylcarbamate.

The compound of formula (II) is an amine and can form salts, for example addition salts with organic or inorganic acids when it reacts with the suitable acids, by conventional methods. Examples of said salts include hydrochloride, hydrobromide, sulfate, methanesulfonate, phosphate, nitrate, benzoate, citrate, tartrate, fumarate, maleate, etc. Said salts can be obtained by conventional methods by reacting the free amine with the acid in question. In a particular embodiment, said salt is hydrochloride or tartrate.

In another aspect, the present invention relates to a method, hereinafter method of the invention [2], of obtaining a phenyl carbamate of formula (I): 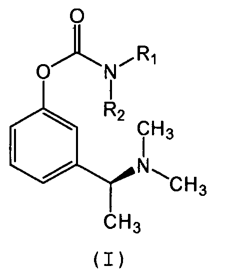 R₁ is C₁-C₅ lower alkyl or benzyl;
R₂ is methyl, ethyl or propyl; or
its solvates, hydrates or pharmaceutically acceptable salts,
which comprises:
a) reacting a compound of formula (VII) with methylamine in the presence of a reducing agent to obtain the compound of formula (VI) by means of reductive amination as shown below: 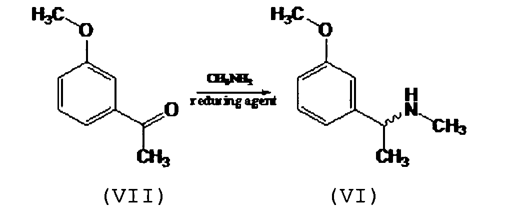
b) subjecting said compound of formula (VI) to a demethylation reaction to obtain a compound of formula (V) 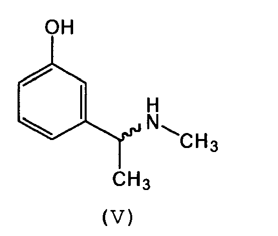
c) resolving the enantiomeric mixture obtained in step b) to obtain the enantiomer of formula (IV) 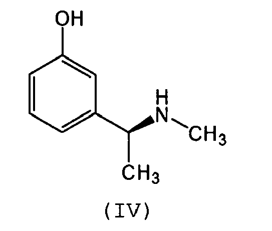
d) reacting said compound of formula (IV) with a compound of formula (III) 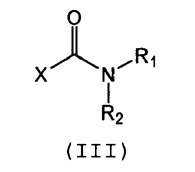 wherein R₁ and R₂ have the previously mentioned meanings and X is a halogen,
   in the presence of a base, to give a compound of formula (II) 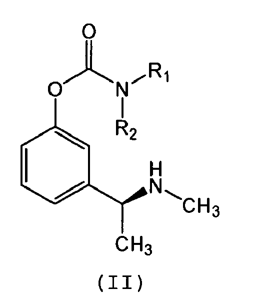 wherein R₁ and R₂ have the previously mentioned meanings; and
e) subjecting said compound of formula (II) to a reductive amination reaction, or alternatively to a methylation reaction by reacting said compound of formula (II) with a methyl halide, to obtain the compound of formula (I).

The first step [step a)] of the method of the invention [2] consists of subjecting a compound of formula (VII) to reductive amination, using to that end methylamine in the presence of a reducing agent, to obtain the compound of formula (VI). Any suitable reducing agent can be used; nevertheless, in a particular embodiment a hydride, such as sodium cyanoborohydride, sodium borohydride together with titanium tetraisopropoxide (Ti(OiPr)₄), sodium borohydride together with acetic acid, etc., or other reducing agents, for example, hydrogen together with a metal catalyst, such as Pd, Ni, etc., can be used as a reducing agent. In a preferred embodiment of the present invention, methylamine, sodium borohydride and Ti(OiPr)₄ are used. The solvents used are, in general, alcohols, for example alcohols with one to five carbon atoms, preferably ethanol. The reaction can be carried out at a temperature comprised between -20°C and 40°C, preferably between 15°C and 25°C.

Once the compound of formula (VI) with the monomethylated amine is obtained, a demethylation reaction takes place in the presence of an inorganic acid. Although virtually any inorganic acid can be used, in a particular embodiment hydrobromic or hydriodic acid is used; the demethylation reaction is preferably carried out using aqueous hydrobromic acid. It should be mentioned that the demethylation reaction of compound (VI) having a monomethylated amine occurs with a higher yield than when an equivalent starting compound with a dimethylated amine is used.

The compound of formula (V) has a chiral carbon and is found in the form of mixtures of its enantiomers or enantiomeric mixtures. As it is used in this description the term "mixtures of enantiomers" or "enantiomeric mixtures" includes both racemic mixtures and the mixtures rich in any one of the enantiomers. For the purpose of obtaining the desired enantiomer [compound of formula (IV)], the compound of formula (V) is subjected to a resolution process which can be carried out by any conventional method, for example using chiral chromatographic columns or by means of fractional crystallization of salts of the enantiomers corresponding with the suitable chiral acids. In a particular embodiment, the (S) enantiomer of the compound of formula (V) is separated by means of optical resolution by treating the mixture of enantiomers with bromocamphorsulfonic acid as described in J. Chem. Soc. 1932, 2513*.* The obtained salt can be recrystallized as many times needed until obtaining the (S) enantiomer of the compound of formula (V) with the desired purity.

Steps d) and e) of the method of the invention [2] correspond to steps a) and b) of the method of the invention [1] and have been described above.

Alternatively to the method of the invention [2] described above, to obtain the compound of formula (I), the order of the reactions corresponding to steps b) (demethylation) and c) (resolution) could be inverted, i.e. the resolution step for resolving the compound of formula (VI) could be carried out first to obtain the desired enantiomer, and then the demethylation reaction for demethylating said enantiomer obtained could be carried out to provide the optically active intermediate of formula (IV).

Therefore, in another aspect the present invention relates to an alternative method, hereinafter method of the invention [3], of obtaining a phenyl carbamate of formula (I): 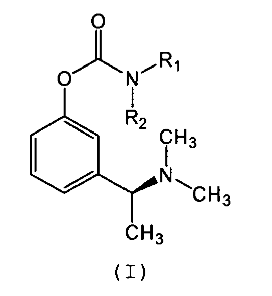 wherein
R₁ is C₁-C₅ lower alkyl or benzyl; and
R₂ is methyl, ethyl or propyl;
its solvates, hydrates or pharmaceutically acceptable salts, which comprises:
a) reacting a compound of formula (VII) with methylamine in the presence of a reducing agent to obtain the compound of formula (VI) by means of reductive amination as shown below: 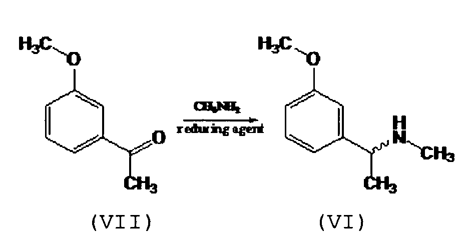
b) resolving the enantiomeric mixture obtained in step a) to obtain the enantiomer of formula (VIa) 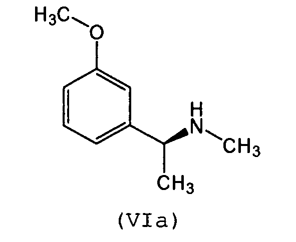
c) subjecting said enantiomer of formula (VIa) to a demethylation reaction to obtain the compound of formula (IV) 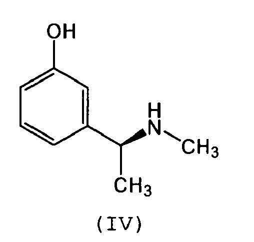
d) reacting said compound of formula (IV) with a compound of formula (III) 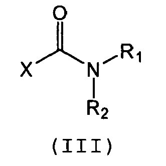 wherein R₁ and R₂ have the previously mentioned meanings and X is a halogen,
   in the presence of a base, to give a compound of formula (II) 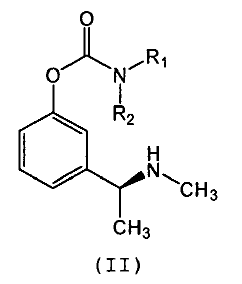 wherein R₁ and R₂ have the previously mentioned meanings; and
e) subjecting said compound of formula (II) to a reductive amination reaction, or alternatively to a methylation reaction by reacting said compound of formula (II) with a methyl halide, to obtain the compound of formula (I).

The first step [step a)] of the method of the invention [3] is common to step a) described in the method of the invention [2], i.e. it consists of a reductive amination in which the reagents used as well as the reaction conditions are the same as those already specified without there being any variation with regard to the previous method.

The compound of formula (VI) obtained after carrying out step a) has a chiral carbon and is therefore in the form of its enantiomeric mixtures.

For the purpose of obtaining the desired enantiomer [compound of formula (VIa)], the compound of formula (VI) is subjected to a resolution process. Resolving an enantiomeric mixture of the compound of formula (VI) can be carried out by means of conventional methods; nevertheless, in a particular embodiment resolving an enantiomeric mixture of the compound of formula (VI) is carried out by means of fractional crystallization of diastereoisomeric salts formed from the reaction of the corresponding enantiomers with a suitable chiral acid.

In a particular embodiment, the chiral (S) enantiomer, i.e. of the compound of formula (VIa), is separated by recrystallizing the corresponding salts with D-(-)-tartaric acid in an alcohol solvent, for example, in an alcohol with one to five carbon atoms, or in an alcohol-water mixture; preferably in methanol.

Under these conditions, a diastereoisomeric salt rich in the desired enantiomer, for example, rich in the enantiomer of formula (VIa) is obtained which, if required, can be subjected to a second recrystallization in an alcohol solvent, preferably methanol, or in an alcohol/water mixture, or to successive recrystallizations until obtaining the desired optical purity.

The desired enantiomer can be obtained by means of resuspending the obtained diastereoisomeric salt in an aqueous-organic medium comprising water and at least one organic solvent, such as dichloromethane or ethyl acetate for example, preferably dichloromethane, and releasing the product by means of neutralization using a base, such as an inorganic base, for example sodium carbonate, sodium hydroxide or potassium hydroxide, among others, preferably sodium hydroxide, until reaching a pH comprised between 7 and 12, preferably between 8 and 9.

In another particular embodiment, the chiral (S) enantiomer, i.e. the compound of formula (VIa), is separated by recrystallizing the corresponding salts with dibenzoyl-L-tartaric acid monohydrate in an alcohol solvent, for example in an alcohol with one to five carbon atoms, or in an alcohol/water mixture; preferably in ethanol or in ethanol/water.

Under these conditions, a diastereoisomeric salt rich in the desired enantiomer, for example, in the enantiomer of formula (VIa) is obtained which, if required, can be subjected to a second recrystallization in an alcohol solvent, preferably ethanol, or in an alcohol/water mixture, for example, ethanol/water, or to successive recrystallizations until obtaining the desired optical purity. Finally, the desired enantiomer can be obtained by releasing the salt in the conditions described for the case of D-(-)-tartaric acid.

Finally, the enantiomer of formula (VIa) is separated from the organic phase and can be isolated in the form of a base, such as an oil, or by forming a suitable acid addition salt, for example hydrochloride, hydrobromide, etc., preferably hydrochloride.

In this case, the enantiomer of formula (VIa), unlike the phenol derivative of formula (IV), can be readily extracted with an organic solvent once the salt formed with the optically active acid is released, whereas the residual acid remains in the aqueous solution.

Resolution of the enantiomeric mixture of the compound of formula (VI) has very good yields and is an additional aspect of the present invention.

The obtained enantiomer [compound of formula (VIa)] is subsequently subjected to a demethylation reaction (step c) to obtain the optically active intermediate [compound of formula (IV)]. This reaction is carried out according to the same method described in step b) of the method of the invention [2], the reagents used as well as the defined reaction conditions being common to both. It is verified by means of measuring the rotatory power that the obtained optical purity is similar to the optical purity obtained with the method of the invention [2].

Steps d) and e) of the method of the invention [3] also correspond to steps a) and b) of the method of the invention [1] and have been described above.

The enantiomeric mixture of the compound of formula (VI) is resolved by means of an original method and it is an additional aspect of this invention. Therefore, in another aspect the invention relates to a method for resolving an enantiomeric mixture of a compound of formula (VI): 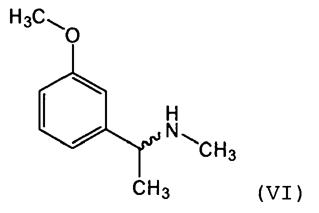 which comprises:
a) forming diastereoisomeric salts by means of reacting the corresponding enantiomers of the enantiomeric mixture with an optically active acid;
b) recrystallizing the diastereoisomeric salts obtained in step a) in an alcohol solvent or in an alcohol solvent/water mixture to obtain a diastereoisomeric salt rich in the desired enantiomer; and
c) obtaining the desired enantiomer by means of:
   c.1) resuspending the diastereoisomeric salt obtained in step b) in an aqueous-organic medium;
   c.2) neutralizing the medium using a base until reaching a pH comprised between 7 and 12; and
   c.3) separating the organic phase and isolating the desired enantiomer in the form of a base or in the form of an acid addition salt.

The optically active acid used can be any optically active acid which forms diastereoisomeric salts with the enantiomers of the enantiomeric mixture of the compound of formula (VI) and which allows separating the desired enantiomer. In a particular embodiment, said optically active acid is D-(-)-tartaric acid or dibenzoyl-L-tartaric acid. The diastereoisomeric salts thus obtained are recrystallized in an alcohol, generally an alcohol with one to five carbon atoms, preferably methanol or ethanol, or in an alcohol solvent/water mixture, for example ethanol/water, to obtain a diastereoisomeric salt rich in the desired enantiomer. The desired enantiomer is then obtained by means of resuspending the previously obtained diastereoisomeric salt rich in the desired enantiomer in an aqueous-organic medium comprising water and at least one organic solvent, for example dichloromethane and ethyl acetate, among others, preferably dichloromethane. The desired enantiomer is then released by neutralization, adding a base until reaching a pH comprised between 7 and 12, preferably, between 8 and 9. Illustrative and non-limiting examples of said bases include inorganic bases, for example sodium carbonate, sodium hydroxide or potassium hydroxide, among others, preferably sodium hydroxide. Finally, the desired enantiomer is obtained from separating the organic phase; said compound can be isolated in the form of a base, such as an oil, or by means of forming a suitable acid addition salt, for example hydrochloride, hydrobromide, etc., preferably hydrochloride. In a particular preferred embodiment the desired enantiomer is the chiral (S) enantiomer, i.e. the compound of formula (VIa) [L-(S)-[1-(3-methoxyphenyl)ethylmethylamine]].

The compound of formula (VIa) can be used to obtain the compound of formula (I) and is an additional aspect of this invention.

Therefore, in another aspect the invention relates to a compound of formula (VIa) 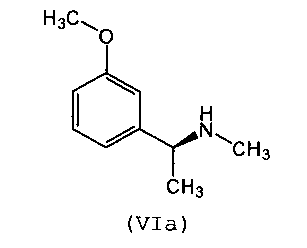 or a salt thereof.

The compound of formula (VIa) is an amine and can form salts, for example addition salts with organic or inorganic acids when it reacts with the suitable acids, by conventional methods. Illustrative examples of said salts include hydrochloride, hydrobromide, sulfate, methanesulfonate, phosphate, nitrate, benzoate, citrate, tartrate, fumarate, maleate, etc., preferably hydrochloride. Said salts can be obtained by conventional methods by reacting the free amine with the acid in question.

Said compound of formula (VIa) can be obtained by means of a method such as the one described above in relation to the method of the invention [3] and has the advantage that it can be readily extracted from an aqueous medium by means of extraction with a suitable organic solvent, for example dichloromethane. Said compound of formula (VIa) can additionally be obtained by means of resolving an enantiomeric mixture of a compound of formula (VI) as described below and which is an additional aspect of this invention.

In another aspect the invention additionally relates to a method of obtaining said compound of formula (VIa) by resolving an enantiomeric mixture of a compound of formula (VI): 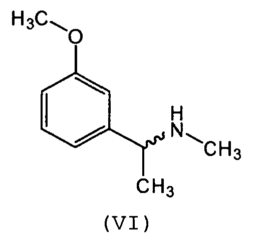 which comprises:
a) forming the diastereoisomeric salts by means of reacting the corresponding enantiomers of the enantiomeric mixture with an optically active acid;
b) recrystallizing the diastereoisomeric salts obtained in step a) in an alcohol solvent or in an alcohol solvent/water mixture to obtain a diastereoisomeric salt rich in the enantiomer of formula (VIa); and
c) obtaining the enantiomer of formula (VIa) by means of:
   c.1) resuspending the diastereoisomeric salt obtained in step b) in an aqueous-organic medium;
   c.2) neutralizing the medium using a base until reaching a pH comprised between 7 and 12; and
   c.3) separating the organic phase and isolating the enantiomer of formula (VIa) in the form of a base or in the form of an acid addition salt.

The practical way to carry out steps a)-c) has been described above in relation to the method for resolving an enantiomeric mixture of a compound of formula (VI). In a particular embodiment, the optically active acid used is D-(-)-tartaric acid or dibenzoyl-L-tartaric acid, diastereoisomeric salts are recrystallized in an alcohol, generally an alcohol with one to five carbon atoms, preferably methanol or ethanol, or in an alcohol solvent/water mixture, for example ethanol/water, to obtain the diastereoisomeric salt rich in the enantiomer of formula (VIa), which is resuspended in an aqueous-organic medium, comprising water and an organic solvent, such as dichloromethane and ethyl acetate, preferably dichloromethane. The enantiomer of formula (VIa) is then released by means of neutralization, adding a base, for example an inorganic base (e.g., sodium carbonate, sodium hydroxide or potassium hydroxide, preferably sodium hydroxide), until reaching a pH comprised between 7 and 12, preferably between 8 and 9. Finally, the enantiomer of formula (VIa) is obtained from separating the organic phase, being able to be isolated in the form of a base, such as an oil, or by means of forming a suitable acid addition salt, for example hydrochloride, hydrobromide, etc., preferably hydrochloride.

The following examples illustrate the invention and should not be interpreted as limiting the scope thereof.

### Example 1

### Obtaining α-3-methoxyphenylethylmethylamine [compound of formula (VI)]

Under inert conditions, 348 g of titanium isopropoxide are slowly added to a solution of 38 g of methylamine in 2.2 L of ethanol previously cooled to 10°C. Then 108 g of 3-methoxyacetophenone are incorporated, maintaining the temperature between 15°C and 20°C, and the reaction mixture is left stirring at room temperature for 10 hours. After this time has passed, 41 g of sodium borohydride are added, in 1 hour, maintaining the temperature between 20°C and 25°C. After 15 hours at room temperature, the reaction is considered to be finished and is processed by adding 500 mL of a 25% ammonia solution in 30 minutes, leaving the reaction mixture stirring for 2 hours. The resulting suspension is filtered and the filtrate is washed with 300 mL of ethanol. The obtained alcohol solution is distilled to a residue at reduced pressure, resuspended with 500 mL of water and extracted 3 times with 300 mL of methylene chloride. The combined methylene chloride extracts are dried with sodium sulfate and distilled at reduced pressure until obtaining a yellow oil yielding 103 g.
¹H NMR: δ 1.15 (d, 3H, CH-**CH₃**); δ 2.15 (s, 3H, NH-**CH**₃); δ 3.6 (q, 1H, **CH**-CH₃) ; δ 3.8 (s, 3H, O-**CH**₃) ; δ 6.75 (d, 1H, Ar-**H**); δ 6.85 (s, 1H, Ar-**H**); δ 6.9 (d, 1H, Ar-**H**); δ 7.2 (t, 1H, Ar-**H**)

### Example 2

### Obtaining 3-[(1-methylamino)ethyl]phenol [compound of formula (V)]

The oil obtained in Example 1 is dissolved in 412 mL of 48% aqueous hydrobromic acid and is then heated under reflux. Once the reaction has ended, it is distilled at reduced pressure until reaching a residue; 200 mL of water are added and it is adjusted to pH 8.7 with 50% sodium hydroxide solution. The resulting suspension is cooled to 0°C and filtered, obtaining a pink solid yielding 72 g.
¹H NMR: δ 1.15 (d, 3H, CH-**CH₃**) ; δ 2.1 (s, 3H, NH-**CH₃**) ; δ 3.4 (q, 1H, **CH**-CH₃); δ 6.55 (d, 1H, Ar-**H**); δ 6.7 (t, 1H, Ar-**H**); δ 6.75 (s, 1H, Ar-**H**); δ 7.2 (t, 1H, Ar-**H**)

### Example 3

### Obtaining L-(S)-3-[(1-methylamino)ethyl]phenol [compound of formula (IV)]

60 g of the hydrochloride of the compound obtained in Example 2 are mixed with 55 g of ammonium D-bromocamphorsulfonate, and the resulting mixture is dissolved in 300 mL of water under reflux. The solution is progressively cooled until a solid begins to crystallize; the solid is filtered and washed. The mother and wash liquors are adjusted until reaching a pH of 8.7 when the base rich in the sought enantiomer is reached. The solid obtained in the form of hydrochloride (23 g) is mixed with 40 g of ammonium L-bromocamphorsulfonate, 120 mL of water are added and it is heated under reflux. When the solution cools, a precipitate is formed which corresponds to the desired salt, which is isolated by filtration. Finally 47 g of salt are obtained, which is recrystallized once more from water. The base is recovered by resuspending the obtained salt in water and adjusting the pH to 8.7, the sought compound precipitating.
[ α ]_{D}: - 68 (c: 5 in pyridine)
Hydrochloride of the compound is obtained by crystallizing in ethanol.
[α ]_{D}: -20 (c: 10 in water)

### Example 4

### Obtaining L-(S)-N-ethyl-3-[1-(methylamino)ethyl]-N-methyl-phenylcarbamate [compound of formula (II)]

2 g of the compound of formula (IV) [Example 3] are added to a suspension of 0.6 g of sodium hydride (60% in mineral oil) in 20 mL of tetrahydrofuran previously cooled between 0 and - 5°C. The resulting suspension is maintained for 45 minutes in this temperature range, a 0.5 M solution of ethylmethylcarbamoyl chloride in toluene (2 equivalents) then being added. The reaction is maintained between 20°C and 25°C until it ends, 20 mL of a 1% sodium hydroxide aqueous solution then being added. The resulting mixture is distilled to dryness at reduced pressure, and the resulting residue is distributed between 20 mL of methylene chloride and 20 mL of 10% hydrochloric acid aqueous solution. 20 mL of methylene chloride are added to the separated aqueous phase and the mixture is adjusted to pH 8.7 using a 10% sodium hydroxide aqueous solution. The separated organic phase dried with sodium sulfate is distilled at reduced pressure until obtaining a yellow oil which is identified as the desired product (2.5 g).
¹H NMR: δ 1.15-1.2 (2*t, 3H, N-CH₂-**CH₃**) ; δ 1.3 (d, 3H, -CH-**CH₃**) ; δ 2.3 (s, 3H, N-**CH₃**) δ 2.96- 3.04 (2*s, 3H, OCN-**CH₃**) ; δ 3.36-3.44 (2*q, 2H, N-**CH₂**-CH₃) ; δ 3.6 (q, 1H, -**CH**-CH₃) ; δ 6.96 (d, 1H, Ar-**H**); δ 7.05 (s, 1H, Ar-**H**); δ 7.10 (s, 1H, Ar-**H**); δ 7.3 (t, 1H, Ar-**H**)

### Example 5

### Obtaining (S)-N-ethyl-3-[1-(dimethylamino)ethyl]-N-methyl-phenylcarbamate [Rivastigmine]

1 g of the oil obtained in Example 4 is dissolved in 10 mL of methanol and 1.4 mL of acetic acid, cooling the mixture between 0°C and -5 °C. 0.32 g of sodium cyanoborohydride are added to this mixture, maintaining the temperature under 0°C. Once the addition has been completed, 0.53 mL of a 37% formaldehyde aqueous solution in 10 mL of methanol are added and it is maintained stirring at room temperature until the end of the reaction. Then 10 mL of a 10% hydrochloric acid aqueous solution are added, the methanol residues are distilled at reduced pressure and extracted with 10 mL of methylene chloride. 20 mL of methylene chloride are added to the separated aqueous phase and the mixture is adjusted to pH 8.7 with sodium hydroxide aqueous solution. The separated organic phase is distilled at reduced pressure until obtaining a yellow oil weighing 0.8 g, and which is identified as the desired product.
¹H NMR: δ 1.15-1.2 (2*t, 3H, N-CH₂-**CH**₃) ; δ 1.3 (d, 3H, -CH-**CH₃**) ; δ 2.2 (s, 6H, N-**CH₃**); δ 2.96- 3.04 (2*s, 3H, OCN-**CH₃**); 3.2 (q, 1H, -**CH**-CH₃) ; δ 3.36-3.44 (2*q, 2H, N-**CH**₂-CH₃) ; δ 6.96 (d, 1H, Ar-**H**); δ 7.05 (s, 1H, Ar-**H**); δ 7.10 (d, 1H, Ar-**H**); δ 7.3 (t, 1H, Ar-**H**)

### Example 6

### Obtaining L-(S)-3-[(1-methylamino)ethyl]phenol [compound of formula (IV)] from L-(S)-[1-(3-methoxyphenyl)ethylmethylamine] [compound of formula (VIa)], obtained by resolving the compound of formula (VI)

### a) Resolution:

100 g of the compound of formula (VI) [Example 1] are dissolved in 394 mL of methanol and 93.8 g of D-(-)-tartaric acid are added. The resulting mixture is heated under reflux and is maintained for 1 hour. The obtained solution is cooled to room temperature, when the precipitation of a solid is observed, and it is cooled to 0°C and filtered. Two additional crystallizations are performed to finally obtain 76 g of a solid in the form of tartaric salt. The desired base is released by dissolving the salt in 760 mL of water and 1,400 mL of methylene chloride and adjusting the pH to 8.7 with an aqueous solution of an inorganic base (NaOH). A colorless oil is obtained from the separated organic phase once the solvent is removed at reduced pressure, which oil corresponds to the desired product (compound of formula VIa). This product can also be isolated as hydrochloride from HCl in ethanol where it crystallizes.
[ δ ]_{D}: -18 (c: 10 in water of the hydrochloride)

### b) Obtaining the compound of formula (IV):

The previously obtained oil was subjected to the same reaction conditions with aqueous BrH as described in Example 2 to obtain the compound of formula (IV) as a solid, with a similar yield. After isolating it as hydrochloride, it was verified that it had the same rotatory power value as described in the literature.

### Example 7

### Obtaining L-(S)-N-ethyl-3-[1-(methylamino)ethyl]-N-methyl-phenylcarbamate [compound of formula (II) wherein R₁ is methyl and R₂ is ethyl]

0.5 g of the compound of formula (IV) [L-(S)-3-[(1-methylamino)ethyl]phenol] are added to 3.3 mL of a 1M solution of potassium tert-butoxide in tetrahydrofuran, previously cooled between 0°C and -5 °C. The resulting suspension is maintained in these conditions for 45 minutes and then a 0.5 M solution of ethylmethylcarbamoyl chloride in toluene (2 equivalents) is added. The reaction is maintained between 20°C and 25°C until depletion of same. The resulting mixture is vacuum-distilled and 20 mL of methylene chloride and 20 mL of 10% hydrochloric acid aqueous solution are added to the residue. 20 mL of methylene chloride are again added to the aqueous phase and it is adjusted to pH 8.7 with 10% sodium hydroxide aqueous solution. The organic phase is dried with sodium sulfate and distilled at reduced pressure until obtaining a yellow oil (0.5 g) which is identified after purification by column chromatography as the desired product.
¹H NMR: δ 1.15-1.2 (2*t, 3H, N-CH₂-**CH₃**) ; δ 1.3 (d, 3H, -CH-**CH**₃) ; δ 2.3 (s, 3H, N-**CH₃**); δ 2.96-3.04 (2*s, 3H, OCN-**CH₃**); δ 3.36-3.44 (2*q, 2H, N-**CH₂**-CH₃) ; δ 3.6 (q, 1H, -**CH**-CH₃) ; δ 6.96 (d, 1H, Ar-**H**); δ 7.05 (s, 1H, Ar-**H**); δ 7.10 (s, 1H, Ar-**H**); δ 7.3 (t, 1H, Ar-**H**)

### Example 8

### Obtaining (S)-N-ethyl-3-[1-(dimethylamino)ethyl]-N-methyl-phenylcarbamate [Rivastigmine]

2.5 mL of 98% formic acid, 0.4 g of sodium formiate and 1 mL of 37% formaldehyde aqueous solution are added to 0.5 g of the compound of formula (II) [Example 7]. The mixture is heated under reflux and maintained at this temperature until the end of the reaction. It is processed by adding 5 mL of methylene chloride and the mixture is adjusted to pH 8.7. The separated organic phase is distilled until obtaining a residue that is purified by column chromatography and is identified as the desired product.
¹H NMR: δ 1.15-1.2 (2*t, 3H, N-CH₂-**CH₃**) ; δ 1.3 (d, 3H, -CH-**CH₃**) ; δ 2.2 (s, 6H, N-**CH₃**); δ 2.96-3.04 (2*s, 3H, OCN-**CH₃**); 3.2 (q, 1H, -**CH**-CH₃) ; δ 3.36-3.44 (2*q, 2H, N-**CH₂**-CH₃); δ 6.96 (d, 1H, Ar-**H**); δ 7.05 (s, 1H, Ar-**H**); δ 7.10 (d, 1H, Ar-**H**); δ 7.3 (t, 1H, Ar-**H**)

### Example 9

### Obtaining L-(S)-[1-(3-methoxyphenyl)ethylmethylamine] (compound VIa) by resolving with dibenzoyl-L-tartaric acid

A suspension of dibenzoyl-L-tartaric acid monohydrate (45.6 g, 0.1212 mol) in ethanol (100 ml) is heated to dissolution. The resulting mixture is heated to reflux and another solution of [1-(3-methoxyphenyl)ethylmethylamine] (compound VI) (20 g, 0.1212) in ethanol (20 ml) is slowly added for 30 minutes. The obtained solution is cooled to 70°C, when the precipitation of a solid is observed. Stirring is maintained at this temperature for two hours, the resulting suspension is slowly cooled until reaching 25°C. The obtained solid is filtered and washed with ethanol to give rise to a white solid weighing 23.15 g, with a molar yield of 36.49%.

Additional crystallization is carried out using seven volumes of an ethanol:water mixture at a 70:30 ratio, respectively, as a solvent. It is finally filtered and dried and 17 g of the salt are obtained, corresponding to a global molar yield of 27%.

The desired base is released by dissolving the salt in dichloromethane and water and adjusting the pH = 12.
[α]_{D}= -65.01 (c= 1 in methanol)

## Claims

1. A method of obtaining a phenyl carbamate of general formula (I): 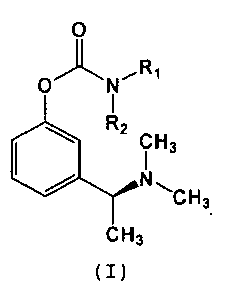 wherein
R₁ is C₁-C₅ lower alkyl or benzyl; and
R₂ is methyl, ethyl or propyl;
its solvates, hydrates or pharmaceutically acceptable salts,
which comprises:
a) reacting a compound of formula (IV): 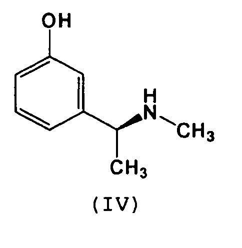 in the presence of a base, with a compound of formula (III) : 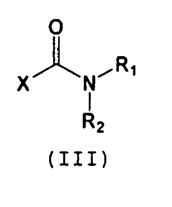 wherein R₁ and R₂ have the previously mentioned meanings and X is a halogen,
to give a compound of formula (II): 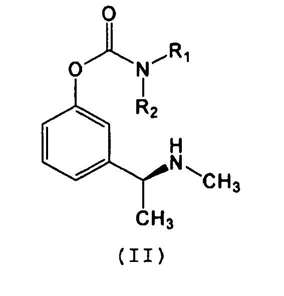 wherein R₁ and R₂ have the previously mentioned meanings; and
b) subjecting said compound of formula (II) to a reductive amination reaction, or alternatively to a methylation reaction by reacting said compound of formula (II) with a methyl halide, to obtain the compound of formula (I).

2. A method according to claim 1, wherein in the obtained compound of formula (I) R₁ is methyl and R₂ is ethyl.

3. A method according to claims 1 or 2, wherein the base used in step a) is NaOH, KOH, NaH, t-BuOK or NaOMe.

4. A method according to claims 1 or 2, wherein reductive amination is carried out in the presence of formaldehyde, sodium cyanoborohydride and acetic acid.

5. A method according to claims 1 or 2, wherein said methyl halide is selected from methyl bromide and methyl iodide.

6. A compound of formula (II): 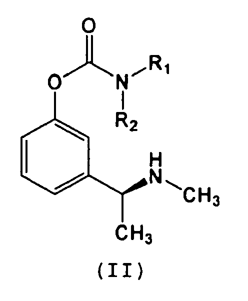 wherein
R₁ is C₁-C₅ lower alkyl or benzyl;
R₂ is methyl, ethyl or propyl;
and its salts.

7. A compound according to claim 6, wherein R₁ is methyl and R₂ is ethyl.

8. A method of obtaining a phenyl carbamate of formula (I) : 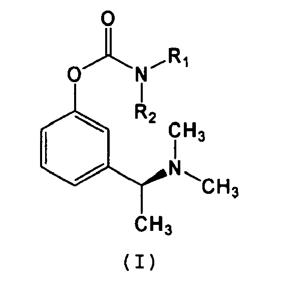 wherein
R₁ is C₁-C₅ lower alkyl or benzyl;
R₂ is methyl, ethyl or propyl;
its solvates, hydrates or pharmaceutically acceptable salts,
which comprises:
a) reacting a compound of formula (VII) with methylamine in the presence of a reducing agent to obtain the compound of formula (VI) by means of reductive amination as shown below: 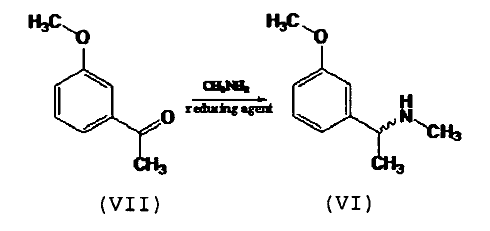
b) subjecting said compound of formula (VI) to a demethylation reaction to obtain a compound of formula (V) 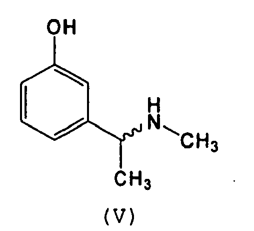
c) resolving the enantiomeric mixture obtained in step b) to obtain the enantiomer of formula (IV) 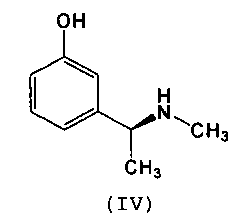
d) reacting said compound of formula (IV) with a compound of formula (III) 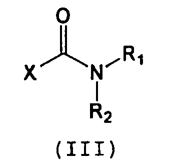 wherein R₁ and R₂ have the previously mentioned meanings and X is a halogen,
in the presence of a base, to give a compound of formula (II) 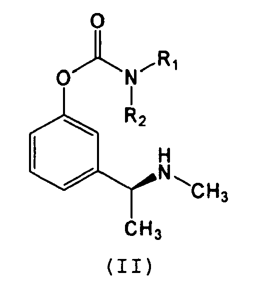 wherein R₁ and R₂ have the previously mentioned meanings; and
e) subjecting said compound of formula (II) to a reductive amination reaction, or alternatively to a methylation reaction by reacting said compound of formula (II) with a methyl halide, to obtain the compound of formula (I).

9. A method of obtaining a phenyl carbamate of formula (I) : 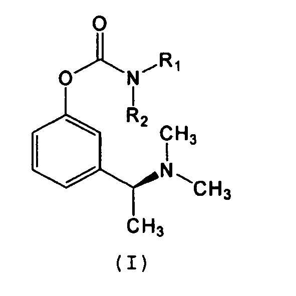 wherein
R₁ is C₁-C₅ lower alkyl or benzyl;
R₂ is methyl, ethyl or propyl;
its solvates, hydrates or pharmaceutically acceptable salts,
which comprises:
a) reacting a compound of formula (VII) with methylamine in the presence of a reducing agent to obtain the compound of formula (VI) by means of reductive amination as shown below: 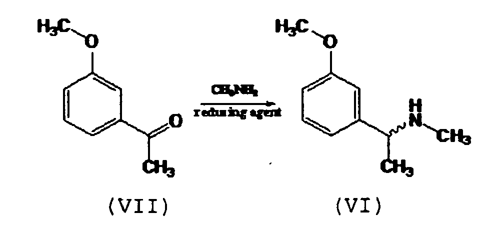
b) resolving the enantiomeric mixture obtained in step a) to obtain the enantiomer of formula (VIa) 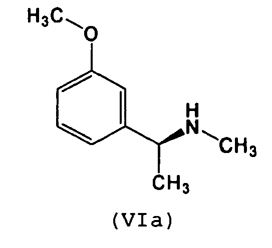
c) subjecting said enantiomer of formula (VIa) to a demethylation reaction to obtain the compound of formula (IV) 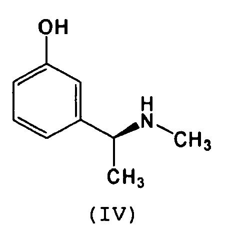
d) reacting said compound of formula (IV) with a compound of formula (III) 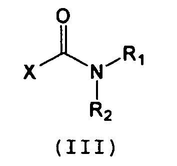 wherein R₁ and R₂ have the previously mentioned meanings and X is a halogen,
in the presence of a base, to give a compound of formula (II) 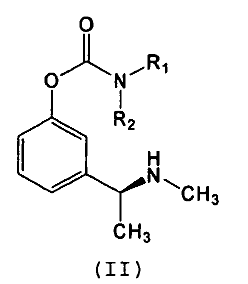 wherein R₁ and R₂ have the previously mentioned meanings; and
e) subjecting said compound of formula (II) to a reductive amination reaction, or alternatively to a methylation reaction by reacting said compound of formula (II) with a methyl halide, to obtain the compound of formula (I).

10. A method according to claim 9, wherein step b) for resolving the enantiomeric mixture is carried out by means of fractional crystallization of diastereoisomeric salts formed from reacting the enantiomers with an optically active acid.

11. A method according to claim 10, wherein said optically active acid is D-(-)-tartaric acid or dibenzoyl-(L)-tartaric acid.

12. A method of resolving the enantiomeric mixture of a compound of formula (VI): 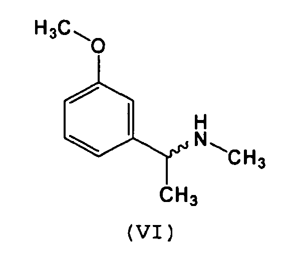 which comprises:
a) forming diastereoisomeric salts by means of reacting the corresponding enantiomers of the enantiomeric mixture with a optically active acid;
b) recrystallizing the diastereoisomeric salts obtained in step a) in an alcohol solvent or in an alcohol solvent/water mixture to obtain a diastereoisomeric salt rich in the desired enantiomer; and
c) obtaining the desired enantiomer by means of:
c.1) resuspending the diastereoisomeric salt obtained in step b) in an aqueous-organic medium;
c.2) neutralizing the medium using a base until reaching a pH comprised between 7 and 12; and
c.3) separating the organic phase and isolating the desired enantiomer in the form of a base or in the form of an acid addition salt.

13. A method according to claim 12, wherein said optically active acid is D-(-)-tartaric acid or dibenzoyl-(L)-tartaric acid.

14. A method according to claim 12, wherein said alcohol solvent is methanol, ethanol or an ethanol/water mixture.

15. A method according to claim 12, wherein said aqueous-organic medium comprises water and an organic solvent.

16. A method according to claim 15, wherein said organic solvent is dichloromethane or ethyl acetate.

17. A method according to claim 12, wherein the enantiomer obtained in step c) is in the form of an acid addition salt, preferably hydrochloride or hydrobromide.

18. A method according to any of claims 12 to 17, wherein the desired enantiomer is the (S) enantiomer of formula (VIa)

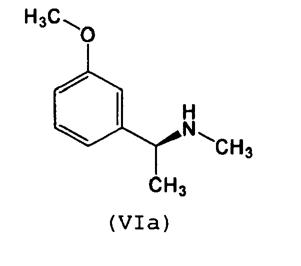

19. A compound of formula (VIa): 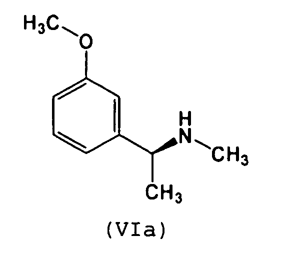 or a salt thereof.

20. A compound according to claim 19 in the form of an acid addition salt.

21. A compound according to claim 20, wherein said acid addition salt is hydrochloride or hydrobromide.

## Patentansprüche

1. Verfahren zur Gewinnung eines Phenylcarbamats der allgemeinen Formel (I): 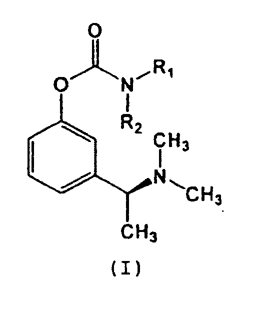 wobei
R₁ ein C₁-5-Niederalkyl oder Benzyl ist; und
R₂ Methyl, Ethyl oder Propyl ist;
seiner Solvate, Hydrate oder pharmazeutisch annehmbaren Salze; umfassend:
a) Umsetzen einer Verbindung der Formel (IV): 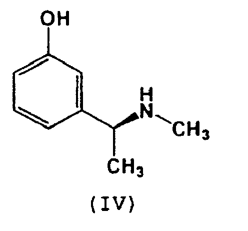 in Gegenwart einer Base mit einer Verbindung der Formel (III): 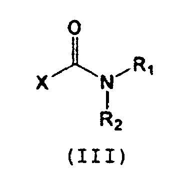 wobei R₁ und R₂ die oben genannten Bedeutungen haben und X ein Halogen ist;
um eine Verbindung der Formel (II) zu erhalten: 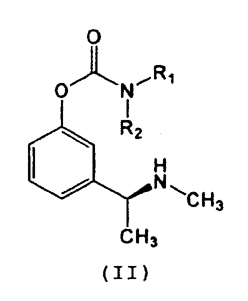 wobei R₁ und R₂ die oben genannten Bedeutungen haben; und
b) Verwendung der Verbindung der Formel (II) zum Durchführen einer reduktiven Aminierungsreaktion oder alternativ dazu einer Methylierungsreaktion durch Umsetzung der Verbindung der Formel (II) mit einem Methylhalogenid, um die Verbindung der Formel (I) zu erhalten.

2. Verfahren gemäß Anspruch 1, wobei in der erhaltenen Verbindung der Formel (I) R₁ Methyl ist und R₂ Ethyl ist.

3. Verfahren gemäß Anspruch 1 oder 2, wobei die Base in Schritt a) NaOH, KOH, NaH, t-BuOK oder NaOMe ist.

4. Verfahren gemäß Anspruch 1 oder 2, wobei die reduktive Aminierung in Gegenwart von Formaldehyd, Natriumcyanoborhydrid und Essigsäure durchgeführt wird.

5. Verfahren gemäß Anspruch 1 oder 2, wobei das Methylhalogenid aus Methylbromid und Methyliodid ausgewählt ist.

6. Verbindung der Formel (II): 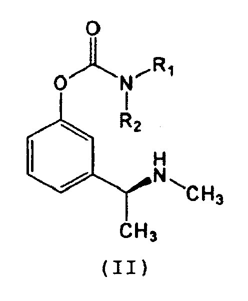 wobei
R₁ ein C₁-C₅-Niederalkyl oder Benzyl ist; und
R₂ Methyl, Ethyl oder Propyl ist;
und ihre Salze.

7. Verbindung gemäß Anspruch 6, wobei R₁ Methyl ist und R₂ Ethyl ist.

8. Verfahren zur Gewinnung eines Phenylcarbamats der allgemeinen Formel (I): 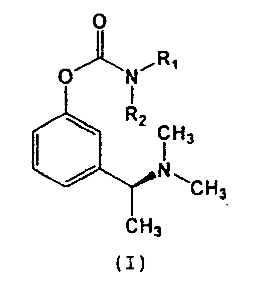 wobei
R₁ ein C₁-C₅-Niederalkyl oder Benzyl ist; und
R₂ Methyl, Ethyl oder Propyl ist;
seiner Solvate, Hydrate oder pharmazeutisch annehmbaren Salze; umfassend:
a) Umsetzen einer Verbindung der Formel (VII) mit Methylamin in Gegenwart eines Reduktionsmittels durch reduktive Aminierung, um die Verbindung der Formel (VI) zu erhalten, wie es im Folgenden gezeigt ist: 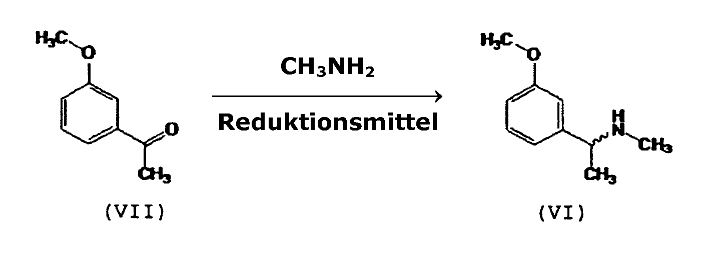
b) Durchführen einer Demethylierungsreaktion mit der Verbindung der Formel (VI), um eine Verbindung der Formel (V) zu erhalten 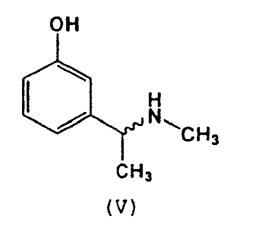
c) Durchführen einer Racematspaltung mit dem in Schritt b) erhaltenen Enantiomerengemisch, um das Enantiomer der Formel (IV) zu erhalten 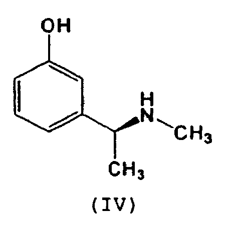
d) Umsetzen der Verbindung der Formel (IV) mit einer Verbindung der Formel (III) 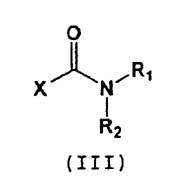 wobei R₁ und R₂ die oben genannten Bedeutungen haben und X ein Halogen ist;
in Gegenwart einer Base, um eine Verbindung der Formel (II) zu erhalten 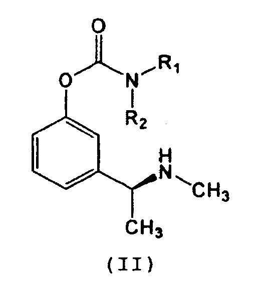 wobei R₁ und R₂ die oben genannten Bedeutungen haben; und
e) Verwendung der Verbindung der Formel (II) zum Durchführen einer reduktiven Aminierungsreaktion oder alternativ dazu einer Methylierungsreaktion durch Umsetzung der Verbindung der Formel (II) mit einem Methylhalogenid, um die Verbindung der Formel (I) zu erhalten.

9. Verfahren zur Gewinnung eines Phenylcarbamats der Formel (I): 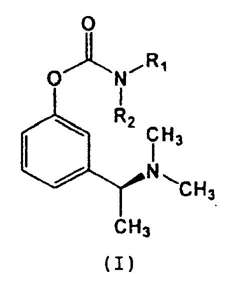 wobei
R₁ ein C₁-C₅-Niederalkyl oder Benzyl ist; und R₂ Methyl, Ethyl oder Propyl ist;
seiner Solvate, Hydrate oder pharmazeutisch annehmbaren Salze; umfassend:
a) Umsetzen einer Verbindung der Formel (VII) mit Methylamin in Gegenwart eines Reduktionsmittels durch reduktive Aminierung, um die Verbindung der Formel (VI) zu erhalten, wie es im Folgenden gezeigt ist: 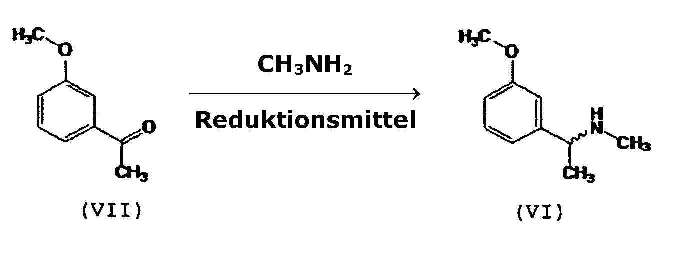
b) Durchführen einer Racematspaltung mit dem in Schritt a) erhaltenen Enantiomerengemisch, um das Enantiomer der Formel (VIa) zu erhalten 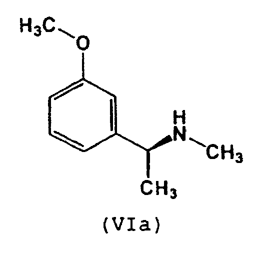
c) Durchführen einer Demethylierungsreaktion mit dem Enantiomer der Formel (VIa), um eine Verbindung der Formel (IV) zu erhalten 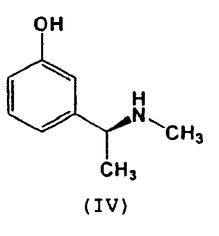
d) Umsetzen der Verbindung der Formel (IV) mit einer Verbindung der Formel (III) 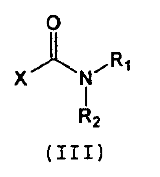 wobei R₁ und R₂ die oben genannten Bedeutungen haben und X ein Halogen ist;
in Gegenwart einer Base, um eine Verbindung der Formel (II) zu erhalten 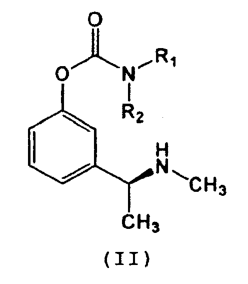 wobei R₁ und R₂ die oben genannten Bedeutungen haben; und
e) Verwendung der Verbindung der Formel (II) zum Durchführen einer reduktiven Aminierungsreaktion oder alternativ dazu einer Methylierungsreaktion durch Umsetzung der Verbindung der Formel (II) mit einem Methylhalogenid, um die Verbindung der Formel (I) zu erhalten.

10. Verfahren gemäß Anspruch 9, wobei Schritt b) zur Racematspaltung mit dem Enantiomerengemisch mittels fraktionierter Kristallisation von diastereomeren Salzen, die durch Umsetzen der Enantiomere mit einer optisch aktiven Säure gebildet werden, durchgeführt wird.

11. Verfahren gemäß Anspruch 10, wobei die optisch aktive Säure D-(-)-Weinsäure oder Dibenzoyl-(L)-weinsäure ist.

12. Verfahren zur Racematspaltung des Enantiomerengemischs einer Verbindung der Formel (VI) 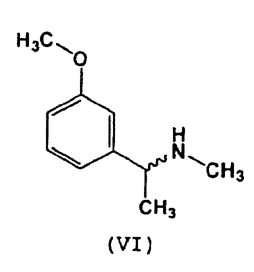 umfassend:
a) Bilden diastereomerer Salze durch Umsetzen der entsprechenden Enantiomere des Enantiomerengemischs mit einer optisch aktiven Säure;
b) Umkristallisieren der in Schritt a) erhaltenen diastereomeren Salze in einem Alkohollösungsmittel oder in einem Alkohollösungsmittel/Wasser-Gemisch, um ein diastereomeres Salz, das an dem gewünschten Enantiomer angereichert ist, zu erhalten; und
c) Gewinnen des gewünschten Enantiomers durch:
c.1) Resuspendieren des in Schritt b) erhaltenen diastereomeren Salzes in einem wässrig-organischen Medium;
c.2) Neutralisieren des Mediums mit Hilfe einer Base, bis ein pH zwischen 7 und 12 erreicht ist; und
c.3) Abtrennen der organischen Phase und Isolieren des gewünschten Enantiomers in Form einer Base oder in Form eines Säureadditionssalzes.

13. Verfahren gemäß Anspruch 12, wobei die optisch aktive Säure D-(-)-Weinsäure oder Dibenzoyl-(L)-weinsäure ist.

14. Verfahren gemäß Anspruch 12, wobei das Alkohollösungsmittel Methanol, Ethanol oder ein Ethanol/Wasser-Gemisch ist.

15. Verfahren gemäß Anspruch 12, wobei das wässrig-organische Medium Wasser und ein organisches Lösungsmittel umfasst.

16. Verfahren gemäß Anspruch 15, wobei das organische Lösungsmittel Dichlormethan oder Ethylacetat ist.

17. Verfahren gemäß Anspruch 12, wobei das in Schritt c) erhaltene Enantiomer in Form eines Säureadditionssalzes, vorzugsweise Hydrochlorids oder Hydrobromids, vorliegt.

18. Verfahren gemäß einem der Ansprüche 12 bis 17, wobei das gewünschte Enantiomer das (S)-Enantiomer der Formel (VIa) 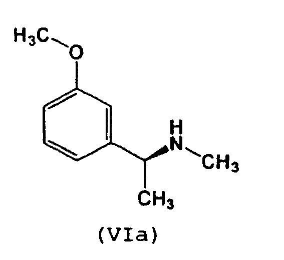 ist.

19. Verbindung der Formel (VIa): 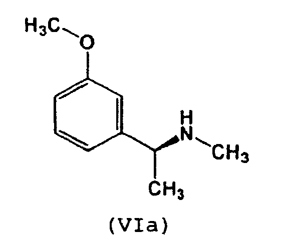 oder ein Salz davon.

20. Verbindung gemäß Anspruch 19 in Form eines Säureadditionssalzes.

21. Verbindung gemäß Anspruch 20, wobei das Säureadditionssalz das Hydrochlorid oder Hydrobromid ist.

## Revendications

1. Procédé d'obtention d'un carbamate de phényle de formule générale (1) : 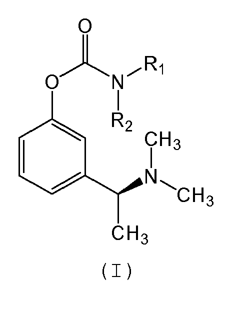 dans laquelle
R₁ est un alkyle inférieur de C₁ à C₅ ou un benzyle ; et R₂ est un méthyle, un éthyle ou un propyle ;
ses solvates, hydrates ou sels pharmaceutiquement acceptables,
qui comprend :
a) la réaction d'un composé de formule (IV) : 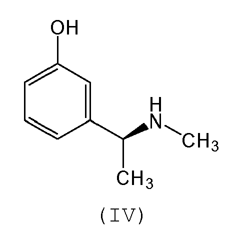 en présence d'une base, avec un composé de formule (III) : 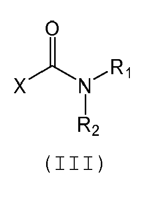 dans laquelle R₁ et R₂ ont les significations mentionnées précédemment et X est un halogène,
pour donner un composé de formule (II) : 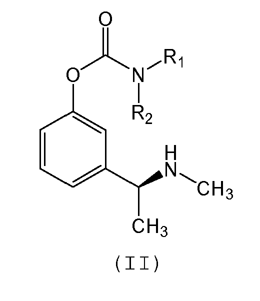 dans laquelle R₁ et R₂ ont les significations mentionnées précédemment ; et
b) la soumission dudit composé de formule (II) à une réaction d'amination réductrice, ou en variante à une réaction de méthylation en faisant réagir ledit composé de formule (II) avec un halogénure de méthyle, pour obtenir le composé de formule (I).

2. Procédé selon la revendication 1, dans lequel dans le composé obtenu de formule (I) R₁ est un méthyle et R₂ est un éthyle.

3. Procédé selon les revendications 1 ou 2, dans lequel la base utilisée à l'étape a) est NaOH, KOH, NaH, t-BuOK ou NaOMe.

4. Procédé selon les revendications 1 ou 2, dans lequel l'amination réductrice est réalisée en présence de formaldéhyde, de cyanoborohydrure de sodium et d'acide acétique.

5. Procédé selon les revendications 1 ou 2, dans lequel ledit halogénure de méthyle est sélectionné parmi le bromure de méthyle et l'iodure de méthyle.

6. Composé de formule (II) : 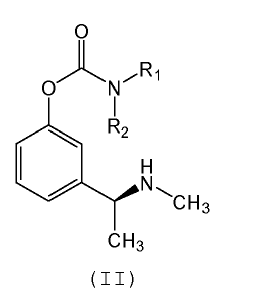 dans laquelle
R₁ est un alkyle inférieur de C1 à C₅ ou un benzyle ; R₂ est un méthyle, un éthyle ou un propyle ;
et ses sels.

7. Composé selon la revendication 6, dans lequel R₁ est un méthyle et R₂ est un éthyle.

8. Procédé d'obtention d'un carbamate de phényle de formule (I) : 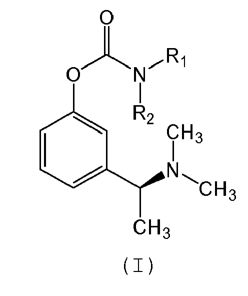 dans laquelle
R₁ est un alkyle inférieur de C₁ à C₅ ou un benzyle ;
R₂ est un méthyle, un éthyle ou un propyle ;
ses solvates, hydrates ou sels pharmaceutiquement acceptables,
qui comprend :
a) la réaction d'un composé de formule (VII) avec une méthylamine en présence d'un agent réducteur pour obtenir le composé de formule (VI) au moyen d'une amination réductrice comme indiqué ci-dessous : 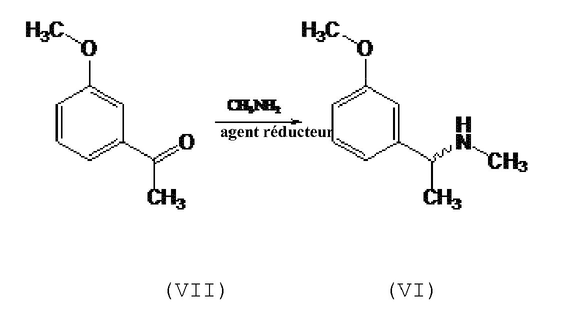
b) la soumission dudit composé de formule (VI) à une réaction de déméthylation pour obtenir un composé de formule (V) 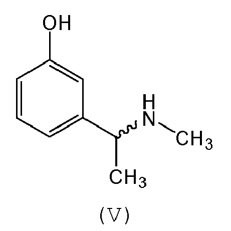
c) le dédoublement du mélange énantiomérique obtenu à l'étape b) pour obtenir l'énantiomère de formule (IV) 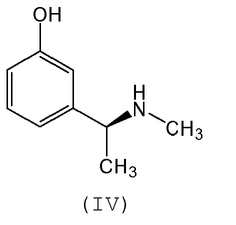
d) la réaction dudit composé de formule (IV) avec un composé de formule (III) 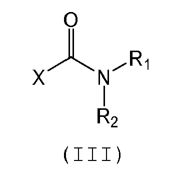 dans laquelle R₁ et R₂ ont les significations mentionnées précédemment et X est un halogène,
en présence d'une base, pour donner un composé de formule (II) 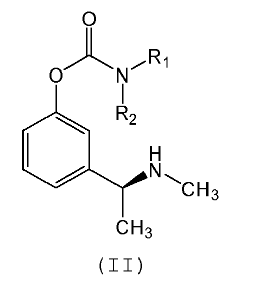 dans laquelle R₁ et R₂ ont les significations mentionnées précédemment ; et
e) la soumission dudit composé de formule (II) à une réaction d'amination réductrice, ou en variante à une réaction de méthylation en faisant réagir ledit composé de formule (II) avec un halogénure de méthyle, pour obtenir le composé de formule (I).

9. Procédé d'obtention d'un carbamate de phényle de formule (I) : 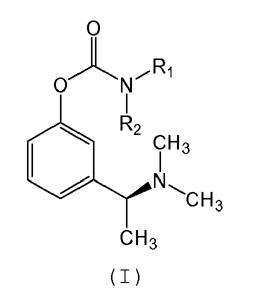 dans laquelle
R₁ est un alkyle inférieur de C₁ à C₅ ou un benzyle ;
R₂ est un méthyle, un éthyle ou un propyle ;
ses solvates, hydrates ou sels pharmaceutiquement acceptables,
qui comprend :
a) la réaction d'un composé de formule (VII) avec une méthylamine en présence d'un agent réducteur pour obtenir le composé de formule (VI) au moyen d'une amination comme indiqué ci-dessous : 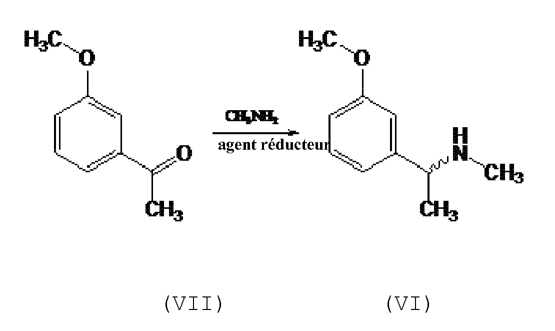
b) le dédoublement du mélange énantiomérique obtenu à l'étape a) pour obtenir l'énantiomère de formule (VIa) 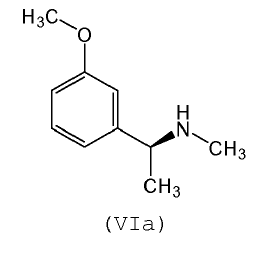
c) la soumission dudit énantiomère de formule (VIa) à une réaction de déméthylation pour obtenir le composé de formule (IV) 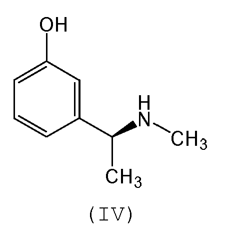
d) la réaction dudit composé de formule (IV) avec un composé de formule (III) 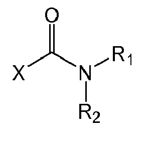 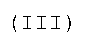 dans laquelle R₁ et R₂ ont les significations mentionnées précédemment et X est un halogène,
en présence d'une base, pour donner un composé de formule (II) 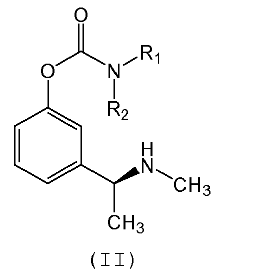 dans laquelle R₁ et R₂ ont les significations mentionnées précédemment ; et
e) la soumission dudit composé de formule (II) à une réaction d'amination réductrice, ou en variante à une réaction de méthylation en faisant réagir ledit composé de formule (II) avec un halogénure de méthyle, pour obtenir le composé de formule (I).

10. Procédé selon la revendication 9, dans lequel l'étape b) de dédoublement du mélange énantiomérique est réalisée au moyen d'une cristallisation fractionnée de sels diastéréoisomériques formés à partir de la réaction des énantiomères avec un acide optiquement actif.

11. Procédé selon la revendication 10, dans lequel ledit acide optiquement actif est l'acide (-)-D-tartrique ou l'acide (L)-dibenzoyl-tartrique.

12. Procédé de dédoublement du mélange énantiomérique d'un composé de formule (VI) : 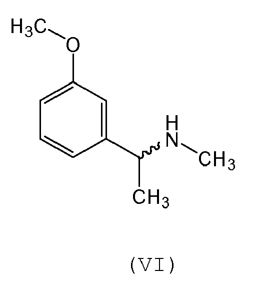 qui comprend :
a) la formation de sels diastéréoisomériques au moyen de la réaction des énantiomères correspondants du mélange énantiomérique avec un acide optiquement actif ;
b) la recristallisation des sels diastéréoisomériques obtenus à l'étape a) dans un solvant à l'alcool ou dans un mélange solvant à l'alcool/eau pour obtenir un sel diastéréoisomérique riche en énantiomère souhaité ; et
c) l'obtention de l'énantiomère souhaité au moyen de :
c.1) la remise en suspension du sel 1 diastéréoisomérique obtenu à l'étape b) dans un milieu aqueux-organique ;
c.2) la neutralisation du milieu à l'aide d'une base jusqu'à obtention d'un pH compris entre 7 et 12 ; et
c.3) la séparation de la phase organique et l'isolement de l'énantiomère souhaité sous la forme d'une base ou sous la forme d'un sel d'addition d'acide.

13. Procédé selon la revendication 12, dans lequel ledit acide optiquement actif est l'acide (-)-D-tartrique ou l'acide (L)-dibenzoyl-tartrique.

14. Procédé selon la revendication 12, dans lequel ledit solvant à l'alcool est le méthanol, l'éthanol ou un mélange éthanol/eau.

15. Procédé selon la revendication 12, dans lequel ledit milieu aqueux-organique comprend de l'eau et un solvant organique.

16. Procédé selon la revendication 15, dans lequel ledit solvant organique est le dichlorométhane ou l'acétate d'éthyle.

17. Procédé selon la revendication 12, dans lequel l'énantiomère obtenu à l'étape c) se présente sous la forme d'un sel d'addition d'acide, de préférence un chlorhydrate ou un bromhydrate.

18. Procédé selon l'une quelconque des revendications 12 à 17, dans lequel l'énantiomère souhaité est l'énantiomère (S) de formule (VIa)

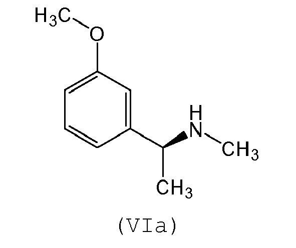

19. Composé de formule (VIa) : 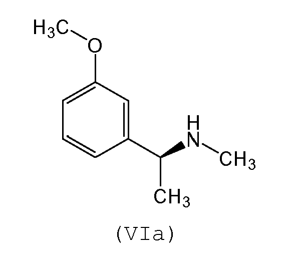 ou sel de celui-ci.

20. Composé selon la revendication 19 sous la forme d'un sel d'addition d'acide.

21. Composé selon la revendication 20, dans lequel ledit sel d'addition d'acide est un chlorhydrate ou un bromhydrate.
